# EUROPEAN PATENT APPLICATION

(11) **EP 4 481 088 A1**
(43) Date of publication of application: **25.12.2024**
(21) Application number: 23180249.7
(22) Date of filing: 20.06.2023
(51) Int. Cl.: C25B 1/26, C25B 11/052, C25B 11/053, C25B 11/061, C25B 11/063, C25B 11/077, C25B 11/081, C25B 11/091, C25B 11/093, C02F 1/467, C25B 1/34, C02F 1/76

(54) **CATALYTIC ANODES AND PROCESSES FOR USE IN ELECTRO-CHLORINATION**

(71) Applicant: Infineum International Limited, Abingdon Oxfordshire OX13 6BB (GB)
(72) Inventor: Schwarz, Andrew, Abingdon, OX13 6BD (GB); Choi, Zungsun, 098632 Singapore (SG); Lee, ChoongHyuk, Daejeon (KR); Choi, Jaeho, Daejeon (KR); Kim, Young, Daejeon (KR); Nam, Ki Tae, 08826 Seoul (KR); Choi, Seungwoo, 08826 Seoul (KR); Park, Sunghak, 08826 Seoul (KR); Seo, Hongmin, 08826 Seoul (KR); Choi, Won II, 08826 Seoul (KR)
(74) Representative: Hart, Richard Joseph

(57) **Abstract**

The present invention relates to catalytic anodes for use in electro-chlorination systems which generate chlorine from aqueous solutions via the chlorine evolution reaction. These anodes comprise an electrically conductive metallurgical catalyst layer essentially comprising crystalline cobalt oxide particles in a matrix of tin and antimony in a defined stoichiometry. The desired characteristics of this metallurgical layer may be realised via a process of preparation employing specific control of reactants and process conditions.

## Description

The present invention relates to catalytic anodes for use in electro-chlorination systems which generate chlorine from aqueous solutions containing chloride ions, via the chlorine evolution reaction. Such chlorine-generating systems are especially useful in the industrial treatment and purification of waste water, such as ballast water treatment systems and industrial water. They may also particularly be utilised in the industrial production of chlorine in the chlor-alkali process.

The chlorine evolution reaction ("CER") is a well-known reaction in industrial chemistry, which finds application in both the large-scale generation of chlorine for capture, and in the generation of chlorine for immediate applications. The chlor-alkali process generates chlorine and sodium hydroxide through the electrolysis of sodium chloride aqueous solutions; the products are recovered and put to subsequent industrial use. Chlorine is also an effective biocide, and consequently electro-chlorination systems are also independently used for water purification based on *in situ* generation of aqueous chlorine and its instant biocidal effect. Microbially-contaminated water can be effectively treated by electro-chlorination, which is deployed in a variety of settings such as industrial waste water and general water sanitation. In addition, ships increasingly carry on-board ballast water treatment systems to treat ballast tank water before discharge. Ships travelling long distances having the potential to introduce alien aquatic organisms into local ecosystems via the discharge of ballast water acquired far away. The invading alien species can cause considerable impact to local ecosystem balance. Electro-chlorination systems driven by electrical generators on board the ship offer an effective and immediate solution for treating such water before discharge.

The effective industrial use of electro-chlorination systems and processes relies on an electrolytic cell that can operate efficiently for extended periods. To promote the generation of chlorine at the anode, it is common in the art to deploy catalytic coatings on the electrolyte-facing surface of the anode to catalyse the chlorine evolution reaction. Such catalytic coatings typically comprise significant amounts of one or more precious metals, of which the most commonly used are iridium and ruthenium, thereby adding significant cost and complexity to anode manufacture. There remains in the art an ongoing need for new catalytic anodes which can more cost-effectively meet the various technical considerations relevant to an industrial or municipal electro-chlorination environment.

In selecting catalytic coatings for electro-chlorination anodes, a variety of technical considerations must be taken into account.

Firstly, the catalyst chosen must be sufficiently selective for the chlorine evolution reaction, so as to reduce the loss of efficiency that results from the competing oxygen evolution reaction, which can otherwise also occur at the anode from the electrolytic splitting of the water in aqueous solutions. Secondly, the catalytic coating must be sufficiently durable to withstand long-term operation and the acidification of electrolyte that can occur with electro-chlorination. Thirdly, the anode coating should be highly conductive, to improve electrical efficiency by avoiding the need for large over-potentials to drive the electrolytic cell. In practice, some degree of overpotential is typically evident in electrolytic cells due to efficiency losses; lower degrees of over-potential, however, provide important relative efficiency gains, and the maintenance of lower over-potentials over extended operation provides a relevant measure of electrode durability and useful lifetime.

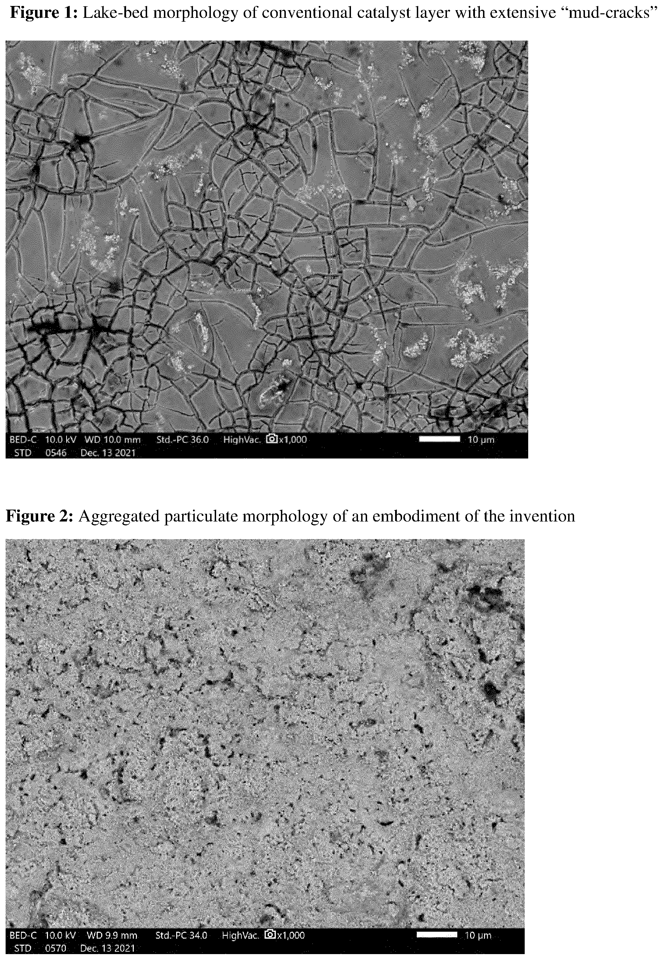

The physical durability of the catalytic coating on the anode can be assessed both by its performance in long-term tests and also by inspection of its morphology, most suitably by scanning electron microscopy. In practice, catalytic anode coatings in the art typically show an outer surface morphology that resembles a dry lake-bed, having the appearance of finely-deposited sediment extensively interrupted by characteristic "mud-cracks" as illustrated in Figure 1. These cracks provide weak points in the catalyst layer, and allow the penetration of electrolyte and reactive species down towards the substrate of the anode, resulting in degradation processes that weaken the adhesion of the catalyst to the substrate and attack the exposed compositions. Loss of electrical efficiency (as evidenced, for example, by increases in over-potential) and loss of catalytic activity typically result and reduce the useful life of the anode, leading to costly replacement.

The catalytic anodes of the first aspect of the invention, being obtainable by the process of the second aspect of the invention, provide an advantageous balance of properties addressing these technical demands of such anodes, whilst avoiding the need for costly precious metals, especially iridium. In particular, the catalyst-coated anode structures defined in the first aspect of the present invention show high catalytic activity and selectivity for the chlorine evolution reaction, show excellent durability in terms of operating lifetime, and show improved resistance to acids formed during operation. They also possess high anode conductivity, resulting in efficient electrical operation without excessive over-potential, maintained over extended running periods.

These advantages of the first aspect of the present invention result from the deployment of an electrically conductive metallurgical catalyst layer overlying the electrolyte-facing surface of the anode, this catalyst layer comprising cobalt oxide crystalline particles consisting essentially of crystalline cobalt oxide having the chemical formula Co₃O₄, within a defined crystalline matrix of oxides of antimony and tin.

The characteristic morphology and chemical composition of this metallurgical catalyst layer is best realised via a process of preparation employing the control of reactants and process conditions further defined in the second aspect of the present invention.

The present invention accordingly provides, in a first aspect, a catalytic anode structure for use in an electro-chlorination system comprising an electrically conductive solid substrate overlaid, on its electrolyte-facing surface, with an electrically conductive metallurgical catalyst layer, this layer comprising cobalt oxide crystalline particles consisting essentially of crystalline cobalt oxide having the chemical formula Co₃O₄, within a crystalline matrix of oxides of antimony and tin;
wherein the cobalt oxide is present in an amount of at least 12.5 atom% of the total metal atom content of the metallurgical catalyst layer; and wherein the overall metallic stoichiometric ratio of the cobalt: total of antimony and tin in the metallurgical catalyst layer is in the range of 2:1 to 9:1.

In the anode of the first aspect, the catalyst composition preferably further comprises one or more noble metals or the oxide compound(s) thereof and, in particular, preferably further comprises ruthenium or an oxide thereof.

Within this specification, the term "atom% of the total metal atom content" in relation to a specified metal in a composition refers to the number of atoms of that metal that are present in the relevant composition, expressed as the percentage of the total number of atoms of metallic elements that are present in the relevant composition, and irrespective of whether such metallic elements are present in elemental or compound form.

Within this specification, the term "overall metallic stoichiometric ratio" for particular metallic elements means the stoichiometric ratio of all atoms of those metallic elements present within the composite metallurgical layer (or other composition, where applicable), irrespective of whether such metallic elements are present in elemental or compound form. For example, in a structure containing 15 atom% of antimony and 15 atom% of tin in purely elemental form, the overall metallic stoichiometric ratio for Sb : Sn is 15:15 or 1:1; and in a structure containing 15 atom% of antimony in the form of elemental antimony, 15 atom% of elemental tin, and a further 15 atom% of tin compounded in oxide form, the overall metallic stoichiometric ratio for Sb : Sn is 15 : (15+15) or 1:2.

In a preferred embodiment of the first aspect, the catalytic anode structure consists of an electrically conductive solid substrate overlaid, on its electrolyte-facing surface, with the above-defined electrically conductive metallurgical catalyst layer. In this embodiment the catalyst layer lies directly adjacent to the substrate.

In an alternative preferred embodiment of the first aspect, the catalytic anode structure comprises an electrically conductive solid substrate overlaid, on its electrolyte-facing surface, with a metallurgical interlayer and the above-defined electrically conductive metallurgical catalyst layer. In this alternative embodiment the interlayer lies directly adjacent to the substrate, and the catalyst composition overlays the interlayer to provide the catalytic surface layer. The preferred interlayer in this embodiment is a dimensionally stable anode composition, and most preferably the interlayer compositions hereinafter described.

The outer surface of the metallurgical catalyst layer according to the above-defined composition has the advantageous morphology of a continuous coating of aggregated cobalt oxide particles embedded in a matrix of tin and antimony oxides.

Within this specification, the term "morphology" is used to denote the three-dimensional structure and appearance of the material in question. The morphology can most usefully be identified by scanning electron microscopy as hereinafter described, which provides three-dimensional imaging of the material surface in a way that allows direct visual recognition of its structure and appearance.

Also within this specification, the term "continuous coating" is used to denote a coating that completely covers the underlying electrode material. Preferably, this continuous coating is uninterrupted by significant cracking or other penetrating discontinuities, that weaken its integrity. In particular, the continuous coating of the metallurgical catalyst layer of the invention advantageously does not exhibit the extensive "mud-cracked" sediment morphology of conventional catalytic coatings used in electro-chlorination, but instead provides a continuous surface covering or layer composed of a three-dimensional matrix of aggregated particles, as visible under scanning electron microscopy.

The present invention is based, in part, on the finding that an anode structure having excellent properties for obtaining the CER reaction in electro-chlorination can be advantageously obtained through a catalyst composition based essentially on cobalt oxide. The resulting metallurgical catalyst layer on the anode provides the anode with excellent durability, in terms of operating lifetime, and offers advantageous catalytic activity and selectivity for the chlorine evolution reaction. The metallurgical catalyst layer also offers the anode high resistance to acid attack, and a high conductivity leading to high electrical efficiency and operating performance at low over-potentials for extended periods. The use of a catalyst composition based largely upon cobalt oxide in preference to higher quantities of precious metals also enables this anode performance to be reached at substantially lower costs than conventional catalytic anodes based on iridium and/or ruthenium as the predominant components.

In particular, the cobalt oxide crystalline particles serve as the essential catalytic species of the metallurgical catalyst layer and provide highly selective catalysis for the CER reaction at substantially lower cost than precious-metal based catalyst compositions such as iridium-based compositions. Whilst the essential cobalt oxide catalyst of the present invention can be supplemented by catalytic precious-metal oxides, the cobalt should be present in an amount of at least 12.5 atom% of the total metal atom content of the metallurgical catalyst layer, and thus represents the predominant catalytic species in the catalyst composition. This use of cobalt oxide as the predominant catalyst species is enabled by the combined use therewith of the defined antimony and tin oxide matrix in the catalyst layer, the combination serving to provide a highly selective, durable and efficient cobalt oxide-based catalytic anode for CER having the above-described improved balance of properties.

The aggregated crystalline particles on the outer surface of the metallurgical catalyst layer are also essential for providing a highly effective and available surface area for catalytic activity. In particular, this particulate morphology overcomes the mass transfer limitations of conventional lake-bed sedimentary catalyst layers, enabling the catalytic anodes of the first aspect of the invention to operate a higher efficiency for the chlorine evolution reaction and contributing to the various advantages of the invention.

The efficiency advantage for the catalytic anode of the present invention in the chlorine evolution reaction is particularly demonstrated with aqueous solutions of chloride ions (such as brine) having low salt levels. Increased efficiency at low salt levels provides an electrode with wide suitability for water treatment, for example in municipal water applications or for treating drinking water, in addition to the treatment of waste water and other chlorination applications. The increased efficiency also results in lower levels of unwanted electrolysis by-products such as chlorate and perchlorate ions.

In a preferred form of the catalytic anode of the first aspect, the catalyst composition does not comprise titanium or a compound thereof. Such a titanium-free catalyst composition provides the resulting anode with the additional advantage of high suitability for use in current-reversal cells. Current reversal has become a maintenance-friendly means for removing deposits of metals such as calcium and magnesium, which may otherwise build up on the cathode during extended electrolytic operation, especially for treatment of municipal or residential water, such as swimming pools, in particular at low salt levels. However, reversing the current inside the cell conventionally causes more rapid degradation and damage to the anode catalytic coating, due to the wide swings in pH that occur at the surface of the electrode, and particularly within the cracked surface of a "mud-cracked" catalyst, where the electrolyte may be brine depleted and the competing water-splitting reaction therefore becomes more prevalent. Titanium present in the coating is susceptible to being dissolved away in this environment, leading to a weakening of the catalytic coating on the substrate and a rapid degradation of the coating and performance. The present invention provides an anode that, because of the high efficiency of its catalyst composition and cobalt content, can dispense with the presence of titanium in the catalyst composition and thereby avoid this degradation, leading to a longer anode lifetime, especially in current-reversal environments.

Such a titanium-free catalyst composition also provides the resulting anode with the additional advantage of greater resilience in high salt level processes, the chlor-alkali process. Typically, a chlor-alkali cell features a Teflon or similar membrane positioned between the cathode and anode. The caustic solution inside the cell can cause holes to develop in the membrane, allowing caustic to reach the anode and cause titanium attack and depletion. The present invention provides an anode that, because of the high efficiency of its catalyst composition and cobalt content, can dispense with the presence of titanium in the catalyst composition and thereby avoid this degradation, leading to a longer anode lifetime in this environment.

The formation of the catalytic anode structure of the first aspect of the invention is best realised through a process which adds the catalyst composition under process conditions ideally favourable to the formation of the catalyst layer of correct composition on the anode surface, and results in the desired morphology of the metallurgical catalyst layer.

The present invention accordingly provides, in a second aspect, a process for the preparation of a catalytic anode structure for use in an electro-chlorination system, comprising the following sequential steps:
a) the preparation or obtention of an electrically conductive solid substrate;
b) the application, to the electrolyte-facing surface of the substrate, of an electrically conductive metallurgical catalyst precursor composition, being a composition comprising cobalt oxide crystalline particles consisting essentially of crystalline cobalt oxide having the chemical formula C_{O3}O₄ dispersed in a mixture of compounds of antimony and tin, wherein the cobalt oxide is present in an amount of at least 12.5 atom% of the total metal atom content of the composition, and wherein the overall metallic stoichiometric ratio of the cobalt: total of antimony and tin in the composition is in the range of 20:80 to 80:20;

wherein the process solvent used for applying the metallurgical catalyst precursor composition in step (b) is a mixture of isopropanol and water in the ratio range of 80:20 to 95:5 by volume; and
wherein the resulting structure is thereafter dried, and then heat-treated at a temperature of at least 450°C.

The process of the second aspect is particularly characterised by its use of a particular starting metallurgical composition and preparative conditions, which give rise to the anode structure of the first aspect.

In a preferred embodiment of the second aspect, the process consists of steps (a) and (b), to arrive at a catalytic anode structure consisting of an electrically conductive solid substrate overlaid, on its electrolyte-facing surface, with the above-defined electrically conductive metallurgical catalyst layer. In this embodiment the catalyst layer lies directly adjacent to the substrate.

In an alternative embodiment of the second aspect, in between the process steps (a) and (b), an additional metallurgical composition (c) may be applied to arrive at the catalytic anode structure comprising an electrically conductive solid substrate overlaid, on its electrolyte-facing surface, with a metallurgical interlayer (derived from the additional metallurgical composition (c)) and the above-defined electrically conductive metallurgical catalyst layer. In this alternative embodiment the interlayer lies directly adjacent to the substrate, and the catalyst composition overlays the interlayer to provide the catalytic surface layer.

During application step (b) the specified process solvent, and subsequent drying and heat-treatment of the resulting structure, contribute to the formation of the advantageous morphology and composition of the resulting metallurgical catalyst layer on the anode of the invention.

The final heat-treatment of the resulting structure at a temperature of at least 450°C has importantly been found to enhance the formation of the desired metallurgical catalyst layer, in particular by favouring high selectivity for the chlorine evolution reaction over the oxygen evolution reaction from water splitting. In this regard the second aspect of the invention prefers a final heat-treatment in the range of 450 to 550°C, and especially an optimal final heat-treatment at 500°C, wherein higher selectivity for the chlorine evolution reaction and durability is observed.

The second aspect of the invention also essentially requires the use of a specified process solvent for applying the metallurgical compositions in step (b) this process solvent being a mixture of isopropanol and water in the ratio range of 80:20 to 95:5 by volume, and preferably in the ratio range of 82.5:17.5 to 87.5:12.5 by volume. Most preferably, the process solvent is a mixture of isopropanol and water in the ratio of 85:15 by volume. This composition of process solvent has been found to show advantages over similar alcoholic solvents for solvency of the starting compounds used in the process of the second aspect. In addition, this composition of process solvent has been found to advantageously control the surface morphology of the layer formed by the application of the metallurgical composition, both before and after the final heat-treatment step, and in particular contributes to the aggregated particulate morphology, free of integrity-weakening cracks. This ability to control layer morphology is particularly attributed to the choice of alcohol and the ratio of alcohol to water in the process solvent, and is optimally controlled at the ratio of 85:15 (isopropanol: water).

In a preferred embodiment of the process, applying the metallurgical catalyst precursor composition to a substrate that is heated to the specified temperature range of 55-200 °C during that application process (and preferably heated to that temperature range throughout that application process) has been found to enhance the formation of the desired metallurgical catalyst layer on the anode.

The drying of the resulting structure at the same temperature range of 55-200°C has also importantly been found to enhance the formation of the desired metallurgical catalyst layer, in particular by favouring the formation of the desired morphology of a continuous layer uninterrupted by mud-cracks or other significant penetrating discontinuities.

Further aspects of the present invention are also described hereunder, and include:
an electro-chlorination system comprising the electrolytic anode structure of the first aspect, or obtainable by the process of the second aspect; and
an electrolytic process for the catalytic evolution of chlorine gas from an aqueous solution containing chloride ions, which comprises passing an electrical current through said solution in an electrolytic cell comprising the electrolytic anode structure of the first aspect or obtainable by the process of the second aspect, wherein chlorine gas is evolved from the aqueous solution at the anode.

In a preferred aspect of the electrolytic process, the chlorine gas evolved by the process is used as a biocide to disinfect water, and more especially used to disinfect ballast water or industrial waste water, or municipal or domestic water, such as recreational water in swimming pools and domestic drinking water. In an alternative preferred aspect, the process is a chlor-alkali process for the generation and recovery of chlorine.

The invention will now be described in more detail as follows:

### Brief description of the Figures

This application includes the following figures:
**Figure 1****:** Lake-bed morphology of conventional catalyst layer with extensive "mud-cracks";
**Figure 2****:** Aggregated particulate morphology of an embodiment of the invention;
**Figures 3 and 4****:** Anode lifetime improvement from the combination of cobalt, tin and antimony in the defined stoichiometry in the catalyst precursor composition of the process; and
**Figure 5****:** Anode CER efficiency improvement from the inclusion of tin and antimony in the defined stoichiometry in catalyst precursor composition of the process.

### First aspect of the invention (catalytic anode)

The catalytic anode structure of the first aspect comprises an electrically conductive solid substrate overlaid, on its electrolyte-facing surface, with an electrically conductive metallurgical catalyst layer, this layer comprising cobalt oxide crystalline particles consisting essentially of crystalline cobalt oxide having the chemical formula Co₃O₄, within a crystalline matrix of oxides of antimony and tin; wherein the cobalt oxide is present in an amount of at least 12.5 atom% of the total metal atom content of the metallurgical catalyst layer; and wherein the overall metallic stoichiometric ratio of the cobalt: total of antimony and tin in the metallurgical catalyst layer is in the range of 2:1 to 9:1.

The anode structures of the first aspect of the invention are obtainable by, and preferably obtained by, the process of the second aspect of the invention.

The anode structures of the first aspect, especially when prepared according to the process of the second aspect, advantageously possess the characteristic that the outer surface of the metallurgical catalyst layer has the morphology of a continuous coating of aggregated particles. The presence of occasional cracks or other surface discontinuities or fault lines is not excluded where such imperfections do not penetrate into the depth of the catalyst layer composition or expose the underlying substrate to electrolyte attack. Preferably, however, the anode structures of the first aspect possess a metallurgical catalyst layer having the outer surface morphology of a continuous, uncracked coating of aggregated particles.

The advantageous aggregated particulate morphology of the outer surface of the metallurgical catalyst layer contrasts with the extensively cracked sediment-like surface morphology of conventional catalytic anodes, and can best be visualised by examination under scanning electron microscopy, as illustrated in the accompanying Figures. In each case, the electron micrographs were obtained by using a JSM-IT500 InTouchScope^{™} Scanning Electron Microscope instrument, using the settings stated in the legend of the relevant Figure.

In Figure 1, scanning electron microscopy of the outer surface morphology of a conventional catalysed anode for electro-chlorination reveals a characteristic "mud-cracking" appearance, with extensive deep cracking radiating across the sediment-like surface coating. Such a morphology renders the catalytic surface prone to chemical attack and weakens its structural integrity, reducing its lifetime and usefulness.

In contrast, in Figure 2, scanning electron microscopy illustrates the surface of the preferred catalysed anode of the first aspect, with a continuous uncracked surface layer and aggregated particulate morphology visible at the magnifications shown.

An essential element of the anode structure of the first aspect is the presence of cobalt oxide present in an amount of at least 12.5 atom% of the total metal atom content of the metallurgical catalyst layer. The anode of the present invention provides a means to deploy a high level of cobalt-oxide based catalyst in a form highly selective for electro-chlorination in an anode that provides excellent durability, in terms of operating lifetime of the catalyst on the anode substrate, high resistance to acid attack, and high conductivity, leading to high electrical efficiency and operating performance at low over-potentials. The use of a catalyst composition based largely upon cobalt oxide as the primary catalytic component in preference to precious metals also enables this anode performance to be reached at substantially lower costs than conventional precious metal-based catalysts, such as iridium-based catalytic anodes.

Preferably, in the anode structure of the first aspect, the cobalt oxide is present in the highest amount practical to maximise its catalytic effect whilst obtaining the other advantages of the invention. Thus, it is preferred that cobalt is present in the amount of at least 15 atom% of the total metal atom content of the metallurgical catalyst layer, and more preferably in the amount of at least 20 atom% of the total metal atom content of the metallurgical catalyst layer. Even more preferably, cobalt is present in the amount of at least 30 atom% of the total metal atom content of the metallurgical catalyst layer, and most preferably in the amount of at least 40 atom% of the total metal atom content of the metallurgical catalyst layer. Ideally, cobalt is present in the amount of 50 to 80 atom% of the total metal atom content of the metallurgical catalyst layer, and optimally in the range of 60 to 75 atom% of the total metal atom content of the metallurgical catalyst layer. Such cobalt contents are advantageously achieved by preparing the anode structure in accordance with the process of the second aspect of the invention.

The individual metal contents and overall metallic stoichiometry of the metallurgical catalyst layer can be measured by known techniques using ICP-MS (Inductively coupled plasma mass spectrometry in order to determine the compositional features of the invention.

In the anode structure of the first aspect, it is preferred that the cobalt oxide crystalline particles in the catalyst composition consist of crystalline cobalt oxide having the chemical formula Co₃O₄. The presence of this crystalline form of cobalt oxide may be confirmed by X-ray diffraction or other conventional crystallographic techniques. The C_{O3}O₄ crystalline particles may be sized by direct measurement of their diameters in transmission electron micrograph images, and preferably to range in diameter from 1nm to 20nm, more preferably from 1nm to 10nm, and most preferably from 3 to 7nm in diameter.

In the anode of the first aspect, the catalyst composition preferably further comprises one or more noble metals or the oxide compound(s) thereof and, in particular, ruthenium or an oxide thereof. In keeping with the object of the invention, the amount of any included noble metals shall be small enough as to not reduce the cobalt content of the metallurgical catalyst layer below the level prescribed by the invention, and the amount of noble metal shall preferably not exceed 10 atom%, and more preferably not exceed 5 atom%, of the total metal atom content of the metallurgical catalyst layer.

In a more preferred embodiment, the cobalt oxide crystalline particles in the catalyst composition consist essentially of crystalline cobalt oxide having the chemical formula Co₃O₄, and further consist essentially of ruthenium to the level of at most 5 atom % of ruthenium based on the total metal atom content of the crystalline particles, with at least some of said ruthenium being present in the form of surface decoration on the crystalline particles. In this latter embodiment of the anode, the cobalt oxide crystalline particles in the catalyst composition may further contain crystalline regions wherein, in addition to surface decoration, ruthenium is incorporated within the crystal lattice of the cobalt oxide, or has formed a mixed metal oxide with cobalt, or both.

The limited quantity of ruthenium provided for in these embodiments in association with the cobalt oxide crystalline particles in the catalyst composition provides the advantage of further improving the properties of the metallurgical catalyst layer on the anode, most notably improving the selectivity of the catalyst for the chlorine evolution reaction, and improving durability of the anode, particularly in terms of operational lifetime. The ruthenium may be present in oxide compound form or in metallic elemental form, at least in the case of incorporation within the crystal lattice of the cobalt oxide. The ruthenium associated with the cobalt oxide catalyst in these embodiments may be present in an overall non-stoichiometric proportion, approximating to a mixture of elemental and oxide-bound ruthenium.

However, exceeding the upper limit of 5 atom% for ruthenium content in the crystalline particles of the catalyst composition has been found to result in a reduction on the efficiency of the chlorine evolution reaction, especially for water treatment applications, as evidenced by a decrease in the low salt efficiency. For this reason the ruthenium content associated with the cobalt oxide catalytic particles should be limited to the stated level, and it is not advantageous to increase ruthenium content further in any expectation of further improvement.

In all the above embodiments of the anode of the first aspect, the metallurgical catalyst layer comprises cobalt oxide crystalline particles distributed on the upper surface of the anode structure. It is preferred that the catalyst composition consists essentially of cobalt oxide crystalline particles distributed in a crystalline matrix of antimony and tin oxides, wherein the matrix does not comprise substantial or any amounts of precious metal oxides such as palladium or iridium, other than as provided herein in relation to the present invention.

In particular, in all embodiments of the anode the catalyst composition consists essentially of, cobalt oxide crystalline particles within a crystalline matrix of oxides of antimony and tin; this catalyst composition being present on the electrolyte-facing surface of the anode.

It has surprisingly been found that the overall ratio of cobalt: total of antimony and tin in the metallurgical catalyst layer provides advantageous performance. By maintaining this within the defined range, the durability of the catalytic anode is improved as evidenced by an increase in its useful lifetime of operation. This increase in lifetime provides the anode of the first aspect with highly advantageous performance.

In particular, in all embodiments of the anode the catalyst composition comprises, and preferably consists essentially of, cobalt oxide crystalline particles within a crystalline matrix of oxides of antimony and tin. It is preferred that the overall metallic stoichiometric ratio of cobalt : total antimony and tin in the metallurgical catalyst layer is in the range of 2:1 to 9:1of More preferably, this overall metallic stoichiometric ratio of cobalt : total of antimony and tin in the metallurgical catalyst layer is in the range of 2.5:1 to 6:1, and most preferably in the range of 3:1 to 5:1. Optimally, the metallurgical catalyst layer is in the range of 3:1 to 4:1. It has surprisingly been found that the overall metallic stoichiometric ratio of cobalt: total of antimony and tin in the metallurgical catalyst layer provides advantageous performance. By maintaining this ratio within the above general range, the durability of the catalytic anode is improved as evidenced by an increase in its useful lifetime of operation. This increase in lifetime provides the anode of the first aspect with highly advantageous performance.

On the anode, it is preferred that the compounds of antimony and tin serving as the matrix in the catalyst composition are present in amounts providing the metallurgical catalyst layer with an overall metallic stoichiometric ratio of antimony : tin in the range of 1:5 to 1:200, and preferably in the range of 1:5 to 1:100. In this regard, it has further been surprisingly found that the overall metallic stoichiometric ratio of antimony : tin in the metallurgical catalyst layer provides a further contribution to obtaining optimal anode performance. Maintaining this overall stoichiometric ratio within the above range provides the anode with further improved lifetime of operation, and also improves the CER efficiency of the cell, as evidenced by Faradic Efficiency.

It is preferred that the compounds of antimony and tin are present in amounts providing the metallurgical catalyst layer with a preferred overall stoichiometric ratio of antimony : tin in the range of 1:25 to 1:100, and most preferably in the range of 1:50 to 1:84. Optimally this ratio may be in the range of 1:50 to 1:85, with excellent embodiments at 1:75 and 1:84.

In each of the above embodiments of the anode structure, it is preferred the metallurgical catalyst layer further comprises at least one compound of palladium in an amount providing the layer with an overall palladium content of at most 7 atom% of the total metal atom content of the composite metallurgical layer. The introduction of a limited quantity of palladium to the layer is preferably done via the inclusion of one or more palladium-based compounds in the catalyst composition, or via subsequent doping. The palladium provides a further improvement in the efficiency of the chlorine evolution reaction, particularly for water treatment applications, as evidenced by an increase in low salt efficiency.

However, exceeding the upper limit of 7 atom% for palladium content in the catalyst composition results in no further improvement of the efficiency of the chlorine evolution reaction, especially for water treatment applications, as evidenced by no further increase in low salt efficiency. For this reason, the palladium content associated with the cobalt oxide catalytic particles should be limited to the stated level to avoid unnecessary cost and complexity. It is preferred that overall palladium content is at most 5 atom%, and more preferably at most 3%, of the total metal atom content of the composite metallurgical layer; and optimally the overall palladium content is at most 1.5 % of the total metal atom content of the composite metallurgical layer.

The anode of all embodiments of the first aspect essentially consists of an electrically conductive solid substrate, which substrates are known in the art. This substrate preferably consists essentially of a valve metal, such as titanium, zirconium, niobium and tantalum (and alloys/mixtures containing these metals), and preferably titanium, and most preferably consists essentially of titanium. Mixtures of valve metals may be present in the substrate. Oxides of the valve metal or metals essentially present in the substrate may additionally be observed or produced as artifacts under the conditions of anode manufacture and use, particularly at the surface of the substrate or its interface with the metallurgical catalyst layer, and their presence is not excluded from the substrates of the invention provided that they do not form the primary compositional component of the substrate.

The anode substrate may be produced by means conventional in the art, and may be purchased or manufactured to order and be formed into any shape suitable for eventual application as an anode in an electrochemical cell or system. In particular, the substrate may be formed into meshes, plates and more complex three-dimensional shapes such as cylinders. The substrates may be coated by the composite metallurgical layer on one or more sides depending on their design and arrangement.

In the anode of all embodiments of the first aspect, the distribution of the cobalt oxide-based catalyst composition extends through the depth of the metallurgical catalyst layer. The distribution of metallic elements through the thickness of the structure of the metallurgical catalyst layer in accordance with the present invention can be visualised by imaging using a transmission electron microscope (TEM) according to the mapping technique TEM-EDS (Transmission Electron Microscopy and Energy Dispersive X-ray Spectroscopy).

Using the TEM-EDS technique, the distribution of various essential compositional metals can be imaged directly, and their distribution observed within the thickness of the metallurgical catalyst layer to confirm the presence of an anode structure of the present invention, especially when compared with an overall image obtained by HAADF (High-Angle Annular Dark Field transmission electron microscopy). Using the TEM-EDS mapping technique, each metallic element can be individually visualised within the metallurgical catalyst layer. In particular, the cobalt of the cobalt oxide particles is readily observed in a dense concentration (coating) on the outer surface of the layer, but cobalt is additionally evident at lower density throughout the depth of the layer. The ruthenium, present in the catalyst composition at a low level, accordingly appears at lower density on TEM-EDS in the surface region of higher cobalt catalyst density. Tin is distributed throughout the layer, being present in the antimony-tin matrix of the catalyst composition.

The distribution and concentration of metallic elements throughout the metallurgical catalyst layer can also be measured quantitatively using the technique TOF-SIMS (Time-Of-Flight Secondary Ion Mass Spectrometry). This technique allows the mapping of metal concentration down through the depth of the metallurgical catalyst layer and can also confirm the presence of the structure of the present invention.

In the anodes of the first aspect, it is preferred that the cobalt of the catalyst composition is distributed through the thickness of the metallurgical catalyst layer.

In a further-enhanced embodiment of the anode of the first aspect, the metallurgical catalyst layer further comprises ruthenium oxide distributed throughout, or substantially throughout, the catalyst layer. In particular, it is preferred that the ruthenium oxide be distributed in the catalyst matrix of antimony and tin oxides described hereinbefore. The inclusion of ruthenium oxide throughout or substantially throughout the metallurgical catalyst layer further improves the electrical conductivity of the layer and increases the low-salt efficiency of the anode, and also improves the long-term durability of the anode structure.

In a further, preferred embodiment of the anode of the first aspect, an additional metallurgical layer ("interlayer") may be present directly on the anode substrate to provide a base layer for the overlying catalyst composition.

In one preferred interlayer embodiment, ruthenium oxide may advantageously be applied as an intermediate layer (or "interlayer") between the anode substrate and the catalyst composition. This is preferably applied during the process of the second aspect, with the subsequent heat treatment of the resulting structure leading to migration of the ruthenium oxide interlayer throughout the catalyst layer, resulting in an enhanced conductive pathway through the catalyst layer and other advantages identified above.

In another preferred interlayer embodiment, an interlayer composition may comprise mixtures of metal oxides, preferably those known in the art as dimensionally stable anode compositions. Such mixtures may comprise titanium oxide, tin oxide and/or ruthenium oxide, and may form a discrete interlayer between the anode substrate and the metallurgical catalyst composition, so functioning as a structural base layer rather than a conventional catalyst layer.

In a more preferred form of the above interlayer embodiment, the interlayer of the anode structure comprises titanium or one or more compounds thereof, or tantalum or one or more compounds thereof, or both. Such an interlayer provides a protective layer on the anode substrate, and is thereafter overlaid with the metallurgical catalyst composition of the invention. Optionally, an intermediate layer of ruthenium oxide may be applied between the interlayer comprising titanium and/or tantalum on the substrate and the metallurgical catalyst composition forming the outer layer of the anode structure.

In an alternative preferred embodiment, the anode has a single interlayer comprising ruthenium or one or more compounds thereof, and preferably also titanium or one or more compounds thereof. Such an interlayer obviates the need for a discrete, intermediate layer of ruthenium or ruthenium compound(s), and instead includes the ruthenium or ruthenium compound(s) within the structure of the interlayer below the metallurgical catalyst composition forming the outer layer of the anode structure.

In another alternative embodiment, the anode structure interlayer comprises iridium or one or more compounds thereof in a limited amount, such that the level of iridium does not become prohibitive of meeting the overall objectives of the invention. Thus, the anode structure comprises iridium, in the form of its oxide or other catalytically active species, in an amount providing an overall iridium content of at most 15 atom% of the total metal atom content of the metallurgical catalyst layer, and preferably at most 5 atom%. The iridium may be present in addition to the other interlayer metallurgy defined above, most notably in addition to ruthenium or titanium or tantalum, or mixtures thereof.

In the above interlayer anode structures, the titanium, tantalum, ruthenium or iridium compounds where present preferably comprise oxide compounds, and preferably consist of oxide compounds. Alternatively, the interlayer composition may comprise mixtures of metal oxides which migrate into or intermingle with the overlying catalyst composition, so forming a composite metallurgical layer on the electrolyte-facing surface of the anode.

This additional presence of a defined base composition, preferably comprising ruthenium, tin and/or titanium oxides as a structural base layer for the overlying catalytic composition, imparts structural integrity to support the overlying cobalt-based catalyst composition.

The preferred embodiment of the structural base composition of this alternative interlayer embodiment of the anode of the first aspect comprises metal oxide compounds of ruthenium, tin and titanium, and preferably consists essentially of metal oxide compounds of ruthenium, tin and titanium. The combination of these three metals is important to obtaining the ideal morphology for the structural base composition *in situ* on the anode surface. Other metal oxides or other compounds may additionally present provided they do not adversely affect the base composition morphology, however most preferably the structural base composition consists of metal oxide compounds of ruthenium, tin and titanium.

In particular, the specific combination of ruthenium, tin and titanium provides a base structural composition exhibiting the base morphology of aggregated particles interspersed with voids, and which is consequently also crack-free, providing a resilient base structure onto which the catalyst composition can be applied.

Furthermore, the specific combination of ruthenium, tin and titanium in the structural base composition provides improved durability for the anode of the first aspect of the invention, when compared to analogous base compositions comprising only ruthenium and titanium, or ruthenium and tin. In this regard, the combination of both tin and titanium with ruthenium provides the anode of the invention with improved anode lifetime and improved mechanical adhesion, and further contributes to the advantages of the invention.

The above-described embodiments of the first aspect of the invention may comprise, in addition to the above-described features, one or more further compositional elements for enhancing particular properties of the anode, provided the overall advantages of the invention are still obtained. For example, in the anode structure of any preceding embodiment, the catalyst composition may additionally comprise a precious catalytic metal (in addition to any palladium, if present), and preferably iridium, in the form of its oxide or other catalytically-active species, in an amount providing an overall additional precious metal content, and preferably iridium content, of at most 15atom%, and preferably at most 5 atom% of the total metal atom content of the composition. The optional presence of this small amount of precious metal, and especially iridium, may be favoured from the viewpoint of further enhancing catalytic performance of the anode, but should be limited in accordance with the object of the invention to minimise the use of costly precious metals and rely primarily on the cobalt oxide for catalytic activity. Accordingly, such amounts of precious metals are much lower than are typically used when basing a catalyst composition on such metals.

Alternatively, or in addition, the catalyst composition on the anode of all embodiments may further comprise at least one compound of copper in an amount providing the catalyst composition with an overall copper content of at most 10 atom%, and preferably at most 2 atom%; or at least one compound of aluminium in an amount providing the catalyst composition with an overall aluminium content of at most 30 atom%, and preferably at most 20 atom%; or at least one compound of nickel in an amount providing the layer with an overall nickel content of at most 15 atom% of the total metal atom content of the metallurgical catalyst layer, and preferably at most 10 atom%, or more than one of the above. The additional presence of copper or aluminium or nickel within the catalyst composition provides a further increase in the catalyst selectivity for the chlorine evolution reaction, as measured for example by the low salt efficiency. It is preferred that the copper and/or aluminium and/or nickel is introduced via and resides within the catalyst composition, preferably in the form of one or more oxide compounds.

### Second aspect of the invention (process of preparation)

The second aspect provides a process for the preparation of a catalytic anode structure for use in an electro-chlorination system, comprising the following sequential steps:
a) the preparation or obtention of an electrically conductive solid substrate;
b) the application, directly to the electrolyte-facing surface of the substrate, of an electrically-conductive metallurgical catalyst precursor composition, being a composition comprising cobalt oxide crystalline particles consisting essentially of crystalline cobalt oxide having the chemical formula C_{O3}O₄ dispersed in a mixture of compounds of antimony and tin, wherein the cobalt oxide is present in an amount of at least 12.5 atom% of the total metal atom content of the composition, and wherein the overall metallic stoichiometric ratio of the cobalt : total of antimony and tin in the composition, is in the range of 20:80 to 80:20;

wherein the process solvent used for applying the metallurgical catalyst precursor composition in step (b) is a mixture of isopropanol and water in the ratio range of 80:20 to 95:5 by volume; and
wherein the resulting structure is thereafter dried, and then heat-treated at a temperature of at least 450°C.

The process of the second aspect of the invention obtains the catalytic anode structure defined under the first aspect of the invention, and is the preferred process for preparing the anode of the first aspect.

In a first preferred embodiment of the process, the metallurgical catalyst precursor composition is applied in step (b) directly to the electrolyte-facing surface of the substrate, to obtain an anode wherein the metallurgical catalyst composition lies directly adjacent to the substrate.

In a second preferred embodiment of the process, a metallurgical base composition is applied directly to the electrolyte-facing surface of the substrate prior to step (b) to form a metallurgical interlayer directly adjacent to the substrate surface; and in step (b) the metallurgical catalyst precursor composition is applied to the outer surface of the metallurgical interlayer. In this embodiment, the metallurgical interlayer is preferably a dimensionally stable anode composition, and more preferably an interlayer of one or more of the preferred kinds described above in respect of the first aspect of the invention.

The temperature conditions and process solvent defined in the process of the second aspect are important to obtaining the desired catalytic anode structure.

In the process after drying, the resulting dried structure is heat-treated at a temperature in the range of at least 450°C, and preferably heat-treated at a temperature in the range of 450 to 550°C. More preferably, the resulting dried structure is heat-treated at a temperature in the range of 475 to 525°C, and optimally at a temperature of 500°C.

The process of the invention also requires the use of a specified process solvent for applying the metallurgical composition in step (b), this process solvent being a mixture of isopropanol and water in the ratio range of 80:20 to 95:5 by volume, and preferably in the ratio range of 82.5:17.5 to 87.5 to 12.5 by volume. Most preferably, the process solvent is a mixture of isopropanol and water in the ratio of 85:15 by volume.

This composition of process solvent has been found to show advantages over similar alcoholic solvents for solvency of the starting compounds used in the process of the second aspect. In addition, this composition of process solvent has been found to advantageously control the surface morphology of the layer formed by the application of the metallurgical composition, both before and after the final heat-treatment step, and in particular contributes to obtaining the aggregated particulate morphology free of integrity-weakening cracks. This ability to control layer morphology is particularly attributed to the choice of alcohol and the ratio of alcohol to water in the process solvent, and is optimally controlled at the ratio of 85:15 (isopropanol:water).

Preferably, in the process of the invention, the anode substrate (a) is heated to a temperature in the range of 55-200°C during step (b), and the resulting structure is thereafter dried at a temperature within the same range. Preferably, the heating of the anode substrate to this temperature range is conducted throughout step (b), and the resulting structure is thereafter dried at a temperature within the same range. Optimally, the substrate (a) is heated to a temperature of 100°C throughout step (b), and the resulting structure thereafter dried at that temperature.

In addition, in relation to the second embodiment comprising the formation of an interlayer prior to step (b) of the process, the same heat-treatment and defined process solvent may be used for applying the metallurgical base composition. During application step (b) the specified conditions of heating of the substrate and the defined process solvent, and subsequent drying and heat-treatment of the resulting structure, contribute to the formation of the advantageous morphology and composition of the resulting composite metallurgical layer on the anode of the invention.

In particular, applying the catalyst composition to a substrate that is heated in accordance with the second aspect of the invention has been found to enhance the formation of the desired metallurgical catalyst layer on the anode. The drying of the resulting structure at the same temperature range has also importantly been found to enhance the formation of the desired metallurgical catalyst layer, in particular by favouring the formation of the desired morphology of a continuous layer, uninterrupted by mud-cracks or other significant penetrating discontinuities.

Likewise, the final heat-treatment of the resulting structure at a temperature of at least 450°C has importantly been found to enhance the formation of the desired metallurgical catalyst layer, by favouring high selectivity for the chlorine evolution reaction over the oxygen evolution reaction from water splitting. In this regard the second aspect of the invention prefers a final heat-treatment in the range of 450 to 550°C, and especially an optimal final heat-treatment at 500°C, wherein higher selectivity for the chlorine evolution reaction and mechanical adhesion is observed.

The use in the process of the second aspect of the above-defined temperature conditions and process solvent, in combination with the defined metallurgical content of the starting composition, results in the formation on the anode substrate of a metallurgical catalyst layer having the desired composition and morphology, suitable for use as the anode of the first aspect.

In the process of the second aspect, the electrically conductive metallurgical catalyst precursor composition comprises cobalt oxide crystalline particles of the kind hereinbefore described in relation to the first aspect.

In this regard the cobalt oxide crystalline particles in the catalyst precursor composition consist essentially of crystalline cobalt oxide having the chemical formula Co₃O₄. In one such embodiment, these cobalt oxide crystalline particles may consist of crystalline cobalt oxide having the chemical formula Co₃O₄. The presence of this crystalline form of cobalt oxide may be confirmed by X-ray diffraction or other conventional crystallographic techniques.

In another, more preferred embodiment, the cobalt oxide crystalline particles in the catalyst precursor composition consist essentially of crystalline cobalt oxide having the chemical formula Co₃O₄, and further consist essentially of ruthenium to the level of at most 5 atom % of ruthenium based on the total metal atom content of the crystalline particles, at least some of said ruthenium being present in the form of surface decoration on the crystalline particles. In this latter embodiment, the cobalt oxide crystalline particles in the catalyst precursor composition may further contain crystalline regions wherein, in addition to surface decoration, ruthenium is incorporated within the crystal lattice of the cobalt oxide, or has formed a mixed metal oxide with cobalt, or both.

The limited quantity of ruthenium provided for in these embodiments in association with the cobalt oxide crystalline particles in the catalyst precursor composition provides the advantage of further improving the properties of the metallurgical catalyst layer when applied to the anode in the process of the invention, most notably by improving the selectivity of the catalyst for the chlorine evolution reaction, and improving durability of the anode, particularly in terms of operational lifetime. The ruthenium may be present in oxide compound form or in metallic elemental form, at least in the case of incorporation within the crystal lattice of the cobalt oxide. The ruthenium associated with the cobalt oxide catalyst in these embodiments may be present in an overall non-stoichiometric proportion, approximating to a mixture of elemental and oxide-bound ruthenium.

However, exceeding the upper limit of 5% for ruthenium content associated with the crystalline particles in the catalyst precursor composition has been found to result in a reduction on the efficiency of the chlorine evolution reaction, especially for water treatment applications, as evidenced by a decrease in the low salt efficiency. For this reason the ruthenium content associated with the cobalt oxide catalytic particles should be limited to the stated level, and it is not advantageous to increase ruthenium content further in any expectation of further improvement.

In all the above embodiments the catalyst precursor composition consists essentially of cobalt oxide crystalline particles is distributed in a mixture with other metal compounds comprising antimony and tin that will, upon application via the process of the second aspect, give rise a crystalline matrix of metal oxides of antimony and tin in the finished catalyst composition of the anode of the invention. It is especially preferred that these other metal compounds do not comprise substantial or any amounts of precious metal oxides such as palladium or iridium, other than as provided herein in relation to the present invention.

In this regard, the process of the second aspect is advantageously conducted using as catalyst precursor a composition wherein the cobalt oxide crystalline particles (as described above) are dispersed in a mixture of compounds of antimony and tin, wherein the overall metallic stoichiometric ratio of the cobalt: total of antimony and tin in the composition is in the range of 20:80 to 80:20. This combination of antimony and tin compounds in the composition gives rise, upon application via the process of the second aspect, to a crystalline matrix of antimony and tin oxides in which the crystalline particles of cobalt oxide are aggregated, giving rise to the desired morphology of the metallurgical catalyst layer.

In this preferred embodiment, it has also been found that using molar ratios of cobalt: total of antimony and tin within the range specified for the metallurgical catalyst precursor composition provides for increased durability of operation of the anode structure resulting from the process of the invention, as demonstrated by maintaining a lower over-potential for a longer period, when compared to ratios outside the specified ratio range. Furthermore, the efficiency of the resulting catalytic anode for the chlorine evolution reaction is improved within the specified range of ratios. In these regards, overall metallic stoichiometric ratios of cobalt: total of antimony and tin from 30:70 to 80:20 are preferred, whilst the range of from 60:40 to 40:60 is more preferred, and the ratio of 50:50 is most preferred and optimal, especially for obtaining a resulting anode having high CER efficiency and a longer operating lifetime at low over-potential.

In the above preferred embodiment, it has also been found that the performance of the resulting anode may be further enhanced when prepared via the process of the second aspect by employing a metallurgical catalyst precursor composition wherein the overall metallic stoichiometric ratio of cobalt : total of antimony and tin is controlled as described above, and furthermore wherein the compounds of antimony and tin are present in amounts providing the composition with an overall metallic stoichiometric ratio of antimony : tin of at most 1:10, and preferably of at most 1:20. Additionally controlling the ratio of antimony to tin in the composition in this range through the process of the invention results in an anode having further improved efficiency for the chlorine evolution reaction.

More preferably in this highly advantageous embodiment of the process, the overall metallic stoichiometric ratio of antimony : tin in the metallurgical catalyst precursor composition is at most 1:50 and especially at most 1:100. The optimal overall metallic stoichiometric ratio of antimony : tin in composition (iii) for achieving increased efficiency of the chlorine evolution reaction is 1:200.

It is most preferred that the metallurgical catalyst precursor composition combines the preferred ranges of the ratio of cobalt : total of antimony and tin, and the preferred ranges of the overall metallic stoichiometric ratio of antimony : tin. Such compositions serve as particularly effective metallurgical catalyst precursor compositions in the process of the second aspect of the invention, and lead to the formation of anode structures with particularly effective combinations of efficiency for the chlorine evolution reaction, and having high durability providing longer operating lifetimes at low over-potentials.

Thus, in particular, in the process of the second aspect it is preferred that the metallurgical catalyst precursor composition comprises the cobalt oxide crystalline particles dispersed in a mixture of compounds of antimony and tin, wherein the overall metallic stoichiometric ratio of cobalt : total of antimony and tin is in the range of 20:80 to 80:20 and the overall metallic stoichiometric ratio of antimony : tin is at most 1:10, and preferably at most 1:20. It is more preferred that the metallurgical catalyst precursor composition comprises the cobalt oxide crystalline particles dispersed in a mixture of compounds of antimony and tin, wherein the overall metallic stoichiometric ratio of cobalt : total of antimony and tin is in the range of 40:60 to 60:40 and the overall metallic stoichiometric ratio of antimony : tin is at most 1:50, and preferably at most 1:100. It is most preferred that the metallurgical catalyst precursor composition comprises the cobalt oxide crystalline particles dispersed in a mixture of compounds of antimony and tin, wherein the overall metallic stoichiometric ratio of cobalt: total of antimony and tin is 50:50 and the overall metallic stoichiometric ratio of antimony : tin is at most 1:100, and optimally is 1:200.

These highly-preferred embodiments for the metallurgical catalyst precursor composition may further comprise the preferred embodiments of the cobalt oxide crystalline particles described hereinbefore, in particular cobalt oxide crystalline particles consisting essentially of crystalline cobalt oxide having the chemical formula Co₃O₄; which crystalline particles may more preferably also consist essentially of ruthenium to the level of at most 5 atom % of ruthenium based on the total metal atom content of the crystalline particles, at least some of said ruthenium being present in the form of surface decoration on the crystalline particles. In this latter embodiment, the cobalt oxide crystalline particles in the metallurgical catalyst precursor composition may further contain crystalline regions wherein, in addition to surface decoration, ruthenium is incorporated within the crystal lattice of the cobalt oxide, or has formed a mixed metal oxide with cobalt, or both.

In the alternative embodiment of the process of the second aspect, wherein a base composition is applied directly to the substate prior to step (b), said applied metallurgical base composition advantageously comprises compounds of ruthenium, tin and titanium in amounts providing the composition with the overall metallic stoichiometry Ru_{≥0.3} (Sn+Ti)_{≤0.7}. It is important in the present invention that the ruthenium content of the applied base composition is stoichiometrically at the level of 0.3 or above (corresponding to 30 atom%) relative to the tin and titanium present in the base composition. It has been found that the durability of the resulting structural base layer is much improved when the ruthenium is at or above this level in the composition when applied according to the process of the second aspect. In addition, the ruthenium assists with the overall conductivity of the base structural composition.

In this regard, it is preferred that composition applied prior to step (b) comprises compounds of ruthenium, tin and titanium in amounts providing the composition with the overall metallic stoichiometry Ru_{0.3} (Sn+Ti)_{0.7}. This level of ruthenium is considered optimal in the applied base composition of this embodiment of the process of the invention, giving rise to a particularly efficient and durable anode structure.

The combined presence of tin and titanium in addition to ruthenium in the base composition results in the formation, via its application in the process of the second aspect, of a structural base layer having the desired morphology of aggregated particles and an absence of the mud-cracking behaviour seen in other compositions comprising ruthenium with only titanium or tin alone. In this respect, the minimum level of 30 atom% of ruthenium relative to tin and titanium in the applied base composition also leads to the enhanced formation of the network of aggregated particles in the morphology of the resulting structural base composition, and a lack of cracking behaviour.

The presence of tin in the applied base composition in combination with ruthenium and titanium also importantly contributes to an increase in long-term durability of the anode prepared according to the second aspect, and gives rise to an increase in the mechanical adhesion of the composite metallurgical layer to the anode substrate.

In order to optimise these advantages, it is preferred that the tin and titanium be present in approximately equal quantities within the applied base composition. Most preferably therefore, the base composition applied prior to step (b) in this embodiment comprises compounds of ruthenium, tin and titanium in amounts providing the applied base composition with the overall metallic stoichiometry Ru_{0.3}Sn_{0.35}Ti_{0.35}.

In the above embodiment of the second aspect, whilst minor amounts of other metallic compounds may be present in addition to the essential compounds of ruthenium, tin and titanium it is preferred that the base composition consists essentially of compounds of ruthenium, tin and titanium, and more preferred that the base composition consist of compounds of ruthenium, tin and titanium, preferably in amounts providing the base composition with the overall metallic stoichiometry Ru_{0.3} (Sn+Ti)_{0.7}, and most preferably with the overall metallic stoichiometry Ru_{0.3}Sn_{0.35}Ti_{0.35}.

The metallurgical catalyst precursor composition and, where used, structural base composition for use in the process of the first aspect may each be prepared from compounds known in the art for the purpose of metal oxide catalyst preparation, mixed together in the appropriate ratios according to the teaching of the invention in the presence of the process solvent. Whilst such compounds may include the relevant metal oxide compounds where practical, typically other compounds are used that allow better mixing or suspension and will oxidise under the subsequent heat-treatment of the process to give rise *in situ* to the desired metal oxides.

Thus, it is preferred in the process of the second aspect that the ruthenium, tin, antimony and titanium compounds present in the applied composition(s), each comprise compounds other than oxide compounds; and wherein their heated application in the process, followed by drying and heat-treatment, thermally decomposes these compounds into one or more of their respective metal oxide or mixed metal oxide compounds within the metallurgical layer(s) of the final anode structure.

In this regard, the ruthenium, tin and titanium compounds present in any base composition preferably comprise, and more preferably consist of, their respective halide compounds, and/or complexes with acetylacetone; the tin and antimony compounds in the metallurgical catalyst precursor composition preferably comprise, and more preferably consist of, their respective halide compounds; and the ruthenium where present preferably comprises, and more preferably consists of, one or more of its respective halide compounds or its complex with acetylacetone. Such compounds provide for suitable dispersion of the metals in the compositions used in the process, and subsequently convert to their respective oxides under the conditions of the process of the invention to give rise to the desired anode of the first aspect of the invention.

The starting compositions for the process may additionally comprise other metal compounds bringing further advantages to the resulting anodes, as detailed under the first aspect.

Thus, the starting compositions may additionally comprise at least one compound of palladium, in an amount providing an overall palladium content of preferably at most 7 atom%, and preferably at most 5 atom% based on the total metal atom content of the starting composition. Preferably the starting catalyst precursor composition additionally comprises at least one compound of palladium, in an amount providing an overall palladium content of preferably at most 7 atom%, and preferably at most 5 atom% based on the total metal atom content of the starting composition.

The catalyst precursor composition may additionally comprise at least one compound of copper, or at least one compound of aluminium, or at least one compound of nickel, or more than one of the above, in amounts respectively providing composition (iii) with an overall copper content of at most 10 atom% , and preferably at most 2 atom% based on the total metal atom content of the composition, or an overall aluminium content of at most 30 atom%, and preferably at most 20 atom% based on the total metal atom content of the composition, or an overall nickel content of at most 15 atom%, and preferably at most 10 atom% based on the total metal atom content of the composition, or more than one of the above.

At least one of the starting process compositions may additionally comprise at least one compound of iridium, in an amount respectively providing an overall iridium content of at most 15 atom%, and preferably at most 10 atom%, based on the total metal atom content of the composition.

These additional metallic species may be introduced via their compounds conventionally used in the art for inclusion in metallurgical coatings.

In a further, preferred embodiment the catalyst precursor composition does not comprise any compound of titanium, giving rise through the process of the second aspect to a catalyst composition that is free of titanium having the attendant advantages hereinbefore described.

In the process of the invention, the electrically conductive solid substrate consists essentially of a valve metal, preferably titanium, as detailed under the first aspect of the invention.

In the process of the invention, metallurgical catalyst precursor composition applied in step (b), and any underlying metallurgical interlayer where present, may be sequentially applied by coating techniques known in the art, such as brush, roller or drawdown application, spray coating by means such as electro-spraying or pressure spraying, dip coating and curtain coating. Thicker coatings may be applied here required, by the coating technique making multiple passes over the underlying structure.

Preferably, each metallurgical composition is sequentially applied by brush-coating in one or more passes over the underlying structure, or by spray-coating in one or more passes over the underlying structure. However, other conventional techniques may also be used to apply one or more of the metallurgical compositions.

### Third aspect of the invention (electro-chlorination system)

In a third aspect, the invention provides an electro-chlorination system comprising the electrolytic anode structure of the first aspect, or obtainable by the process of the second aspect as described above.

### Fourth aspect of the invention (electrolytic process)

In a fourth aspect, the invention provides an electrolytic process for the catalytic evolution of chlorine gas from an aqueous solution containing chloride ions, which comprises passing an electrical current through said solution in an electrolytic cell comprising the electrolytic anode structure of the first aspect or obtainable by the process of the second aspect, wherein chlorine gas is evolved from the aqueous solution at the anode.

In a preferred aspect of the electrolytic process aspect, the chlorine gas evolved by the process is used as a biocide to disinfect water, and more especially used to disinfect ballast water or industrial waste water, or municipal or domestic water, such as recreational water in swimming pools and domestic drinking water. In an alternative preferred aspect, the process is a chlor-alkali process for the generation and recovery of chlorine.

### Worked examples of the invention

### 1. Preparative examples

### Preparation of example starting compositions for the process of the invention

1.A Example starting compositions for use in the process were prepared from the following component compounds and liquids, obtainable in the art:
   *Crystalline solid compounds:*
      RuCl_{3.}xH₂O (available as an item of commerce, and obtained from Sigma-Aldrich),
      SnCl_{4.}5H₂O (available as an item of commerce, and obtained from Sigma-Aldrich),
      SbCl₃ (available as an item of commerce, and obtained from Sigma-Aldrich),
      PdCl₂ (available as an item of commerce, and obtained from Alfa Aesar),
      Ru(acac)₃ (available as an item of commerce, and obtained from Sigma-Aldrich; wherein "acac" represents acetylacetone), and
      Crystalline cobalt oxide particles having the chemical formula Co₃O₄, synthesised as below.
   *Liquids:*
      2-propanol (also termed isopropanol, available as an item of commerce, and obtained from Alfa Aesar),
      De-ionized water,
      TiCl₃ solution (TiCl₃ available as an item of commerce, and obtained from Sigma-Aldrich Sigma-Aldrich, dissolved into a 12% solution in aqueous hydrochloric acid).
   1.B Preparation of a base interlayer composition of the process

A preferred base interlayer composition consisting essentially of ruthenium, tin and titanium compounds having the overall metallic stoichiometry Ru_{0.3}Sn_{0.35}Ti_{0.35} for use in the process to form the structural base composition of the composite metallurgical layer was prepared according to the following sequence.

To a 70 mL vial was added RuCl₃.xH₂O (207.6 mg; 1 mmol) and SnCl₄.5H₂O (409.4 mg; 1.17 mmol). These solid materials were dissolved into the 2-propanol (17 ml) by vortexing, until no metal precursors settle out upon standing. To the resulting solution was added 1.5 mL of the TiCl₃ solution (12 % TiCl₃ in hydrochloric acid solution, 1.17 mmol) by dropwise addition with mechanical stirring at 1,000 rpm. In addition, 1.5 ml of de-ionized water was added dropwise into the solution. The resulting solution was aged for 5 hours with mechanical stirring at 1,000 rpm under ambient conditions, to produce a base composition for use in the alternative embodiment of the process of the invention.

### 1.C Preparation of further interlayer composition of the process

A composition consisting essentially of ruthenium dioxide for use in the process to supplement the optional interlayer was prepared according to the following sequence.

The solvent was prepared by mixing 2-propanol (38.7 ml) and de-ionized water (7.8 ml). Then 15.5 ml of the prepared solvent was placed in a 70ml vial, and thereto added 115.8 mg (0.29 mmol) of Ru(acac)₃ and an additional 2 mL of IPA. The as-prepared solution was then sonicated for 20 minutes, to produce an interlayer composition for use in the process of the invention.

### 1.D Preparation of metallurgical catalyst precursor composition of the process

A preferred catalyst precursor composition consisting essentially of crystalline particles of cobalt oxide, for use in the process as the composition to form the catalyst composition of the composite metallurgical layer, was prepared according to the following sequence.
a) Synthesis of C_{O3}O₄ crystalline particles

Co(NO₃)₂ 6H₂O (170 g, 0.584 mols) was dissolved in ethanol (99.9%, 510 ml) in a beaker at room temperature. To this mixture was added 1-heptanol (510 ml) and the mixture was stirred for 5 minutes. The mixture was transferred to a pressure reactor and heated at 160 °C for 7 hours. The resulting mixture was left for 16 hrs to cool to room temperature and for the solid product to settle. The resulting supernatant was decanted, and the resulting solid product washed with isopropyl alcohol (100 ml) three times. Between each washing the product was thoroughly mixed then centrifuged for 1 hour at 8500 rpm. The supernatant was decanted and removed in each case. Isopropyl alcohol (IPA) was added to meet the solids content of 12 wt% (IPA addition was calculated by the resulting density of the solution once mixed). The resulting mixture was dispersed at 2000 rpm utilising a ball mill with 0.5mm ZrO₂ beads for 2 hours at room temperature to yield the 12wt% C_{O3}O₄ nanoparticle solution.

The above-synthesised C_{O3}O₄ crystalline particles were sized by direct measurement of diameters in transmission electron micrograph images, and found to range in diameter from 3nm to 7nm, with a median diameter of 5nm.

### b) Preparation of metallurgical catalyst precursor composition

The solvent was prepared by mixing 2-propanol (38.7 ml) and de-ionized water (7.8 ml). SbCl₃ (44.7 mg; 0.20 mmol) was dissolved in this solvent by vortexing. 5 ml of this as-prepared solution was then diluted with a further 45 ml of the solvent mixture to form a first "antimony solution". Then 15.5 ml of this antimony solution was mixed with SnCl₄.5H₂O(457.8 mg; 1.31 mmol) with vortexing, to form an "antimony-tin solution".

PdCl₂ (4.8 mg; 0,027 mmol) was placed into a 70 ml vial, and the prepared antimony-tin solution then poured into the vial. Additionally, add 1 ml of the C_{O3}O₄ crystalline particles solution (0.438 mmol) prepared in a) above, and 1 ml of 2-propanol, were each added into the solution. Finally, the mixture was sonicated for 20 minutes, to produce the precursor composition for use in the process of the invention, comprising C_{O3}O₄ crystalline particles incorporating palladium to the level of 1atom% in a solution of antimony and tin compounds wherein the molar ratio of antimony: tin was 1 : 247 and the overall metallic stoichiometric ratio of cobalt : total of antimony and tin was 50:50.

### Preparation of catalytic anode of the invention, via the process of the invention

### Embodiment 1: catalyst precursor composition directly applied to anode substrate

A titanium mesh was sourced as the anode substrate and, after cleaning, heated to the temperature of 100°C using a heating plate. Whilst at this temperature, the above-prepared composition from 1D was applied to the hot substrate surface by brush-coating. The resulting coated sample was held at 100°C for 1 minute the substrate with turned over and the reverse side coated again the substrate was held at 100°C for 1 minute. The coated sample was then transferred to a 500°C oven for 5 minutes. The coated sample was then transferred back to the to the hotplate at 100°C and the coating and heat treatment steps repeated. This brush-coating of the substrate surface with this composition was repeated four additional times, resulting in a total of five brush passes over the surface being coated.

### Embodiment 2: interlayer composition directly applied to anode substrate and overlaid with catalyst precursor composition

An anode according to this embodiment of first aspect of the invention was prepared according to the process of the second aspect of the invention, as follows.

A titanium mesh was sourced as the anode substrate and, after cleaning, heated to the temperature of 100°C using a heating plate. Whilst at this temperature, the above-prepared base composition from 1.B was applied to the hot substrate surface by brush-coating. The resulting coated sample was held at 100°C for 1 minute the substrate with turned over and the reverse side coated again the substrate was held at 100°C for 1 minute. The coated sample was then transferred to a 500°C oven for 5 minutes. The coated sample was then transferred back to the to the hotplate at 100°C and the coating and heat treatment steps repeated. This brush-coating of the substrate surface with this composition was repeated four additional times, resulting in a total of five brush passes over the surface being coated.

The resulting structure was then further brush-coated with above-prepared interlayer composition from 1.C in the same manner as described above, a total of five brush passes being made over the surface. The resulting structure was thereafter further brush-coated with the above-prepared catalyst precursor composition from 1.D, a total of 10 brush passes being made over the surface with this catalyst precursor composition. After these coating steps, the resulting coated structure was dried at the same temperature.

The dried resulting structure was thereafter finally heat-treated in an oven for 1 hour at 500°C, followed by slow cooling within the oven over a period of 8 hours, to obtain the anode structure of the invention.

### Embodiment 3: Anode substrate already coated with conventional dimensionally stable anode composition directly, overlaid with catalyst precursor composition

A commercially available anode structure pre-coated with a conventional dimensionally stable anode composition was thereafter coated with a ruthenium oxide interlayer prepared in line with section 1.C above, and thereafter with the metallurgical catalyst precursor composition prepared in line with Section 1.D above, according to an application process analogous to that used for Embodiment 2 above.

The result was an anode structure bearing a multilayer coating wherein the base layer is a dimensionally stable anode composition, an intermediate layer is ruthenium oxide, and the surface layer is a metallurgical catalyst layer according to the invention.

### 2. Performance examples

The performance and advantages of the anode of the invention were demonstrated using the following experimental techniques.

### Chlorine Evolution Reaction (CER) Efficiency Test:

The Faradic Efficiency of the electrolytic cell provides an important measure of the efficiency of the catalysed anode for the CER reaction.

An electrolytic cell was set up in which a counter-electrode (cathode) and working electrode (anode according to the invention) were deployed as follows.

A 5 × 15 cm² Ti mesh counter electrode was etched with 2.0 M sulfuric acid at 60 °C for 1 hour until the etching solution turned pale purple. The etched Ti mesh was then sonicated several times with 95 % purity ethanol for 10 min. The rinsed Ti mesh was dried by blowing nitrogen gas. The dried counter electrode was then masked with silicon tape and electroplating tape to leave 5 × 5 cm² area.

An anode prepared according to the invention was also masked with silicon tape and electroplating tape to leave 1 × 5 cm² area. Masking the electrodes helped to concentrate the current into confined areas of the electrodes exposed to the electrolyte and hence to increase current density and accelerate the test.

The electrolytic cell was assembled by attaching the masked counter electrode to one side of the electrode frame, and attaching the working electrode to the opposite side of the frame. At the bottom end of both electrodes, part of the frame kept the gap between the electrodes at 5 mm.

The above test cell was configured and operated under the following condition:
a) 500 ml of 20 mM NaCl solution was poured into a 500 ml volume beaker. The above-assembled electrode cell was placed within the beaker and two electric wires connected to the working electrode and the counter electrode respectively.
b) The solution was stirred at 300 rpm using a magnetic stirring bar. An attached potentiostat was then operated in the chrono-potentiometry mode at 40 mA/cm² for 1 minute under the 300 rpm stirring condition.
c) After the 1 minute of operation, the active chlorine concentration was measured and compared to the theoretical concentration resulting from the initial chloride ion concentration to determine the Faradic Efficiency (FE) of the cell incorporating the working electrode (anode of the invention).

The active chlorine concentration was measured by one of two interchangeable methods, described below:

### Active chlorine concentration measurement by DPD colorimetry

In this method, the DPD vial was washed with DI water until an initial test confirmed that no residual active chlorine exists in the test vial.

After the 1 minute of operation of the electrolytic cell, the solution was stirred at 600 rpm for 1 minute to make a fully homogeneous test solution. Then 1 ml of this well-mixed solution wass diluted to one tenth with 20 mM NaCl solution within the DPD vial to give a test solution volume of 10 ml).The DPD reagent was then added to the DPD vial and the vial vortexed for 10 seconds. The active chlorine concentration was then measured with the DPD pocket colorimeter, and the Faradic efficiency (FE) calculated by dividing the amount of genereted active chlorine by the theoretically produced amount of active chlorine.

### Active chlorine concentration measurement by iodometric titration

After the 1 minute of operation of the electrolytic cell, the solution was stirred at 300 rpm to make a homogeneous solution. Then 1.6 g KI (potassium iodide) was added to the beaker containing the solution. After the addition of KI, the solution turned pale yellow. Upon the addition of 4 ml of glacial acetic acid to the solution, the solution turned pale orange. After the addition of 4 ml of starch solution into the beaker, the solution turned dark blue. The dark blue solution was then titrated with 0.02 N sodium thiosulfate until the solution turned colorless, and the amount of generated active chlorine estimated from the titrated amount of added sodium thiosulfate. The Faradic Efficiency was again calculated by dividing the amount of generated active chlorine by the theoretically produced amount of active chlorine.

### Chlorine Evolution Reaction (CER) Lifetime Test:

The Faradic Efficiency of the electrolytic cell measured regularly over long-term testing provides an important demonstration of the lifetime of the catalysed anode, and hence the durability of the anode for the CER reaction. A rapid increase in FE signals deterioration of the anode and indicates it is reaching the end if its durability for CER.

An electrolytic cell with counter and working electrodes was configured as described above. The working electrode (anode) was masked to 1 cm² to concentrate the current density for test acceleration purposes. The masking was done by applying silicon tape, overlaid by electroplating tape. The top of the working electrode was also ground back to the metal substrate before being connected.
a) 1 litre of 0.6 M NaCl solution was poured into a beaker surrounded by a cooling jacket, which maintained the test solution at 15°C. After allowing 1 hour for the temperature to stabilize, the above-assembled electrode cell was placed within the beaker, and two electric wires connected to the working electrode and the counter electrode respectively.
b) The solution was stirred at 300 rpm using a magnetic stirring bar. An attached potentiostat was then operated in the chrono-potentiometry mode at
c) During operation, the potential of the cell was repeatedly measured and plotted over time, to report the potential for CER over the duration of the lifetime test, and identify the deterioration point at which the potential rapidly increased and the useful life of the anode was exceeded.

### Mechanical adhesion test

The mechanical adhesion of the metallurgical surface layer of the anode was assessed by the following method. A piece of 3M electroplating tape was placed over a portion of the working electrode (anode under test) left unmasked by the preparation as detailed above. The tape was eased into tight contact with the coated electrode surface using gentle pressure with a pipette tip. The tape was thereafter peeled off, and the degree to which a residue of the metallurgical coating remained on the adhesive surface of the peeled-away tape was visually assessed as a measure of the strength of the coating's adhesion to the electrode substrate, with greater residue on the removed tape indicating lower residual adhesion to the substrate.

### Acid stability test

A solution of 5% by weight hydrochloric acid was prepared by diluting concentrated hydrochloric acid to the requisite amount with de-ionised water. The anode under test was cut to a size of 2 cm × 1 cm and placed in a 10 ml vial, to which was added 5 ml of the 5% hydrochloric acid solution, with the anode sample fully submerged. The sample and colour of the acid solution was observed over time.

### 2.1 Performance of the catalyst composition of the invention

The importance of catalyst chemical composition on performance is illustrated below.

### 2.1(a) Inclusion of tin and antimony oxide matrix in the catalyst composition

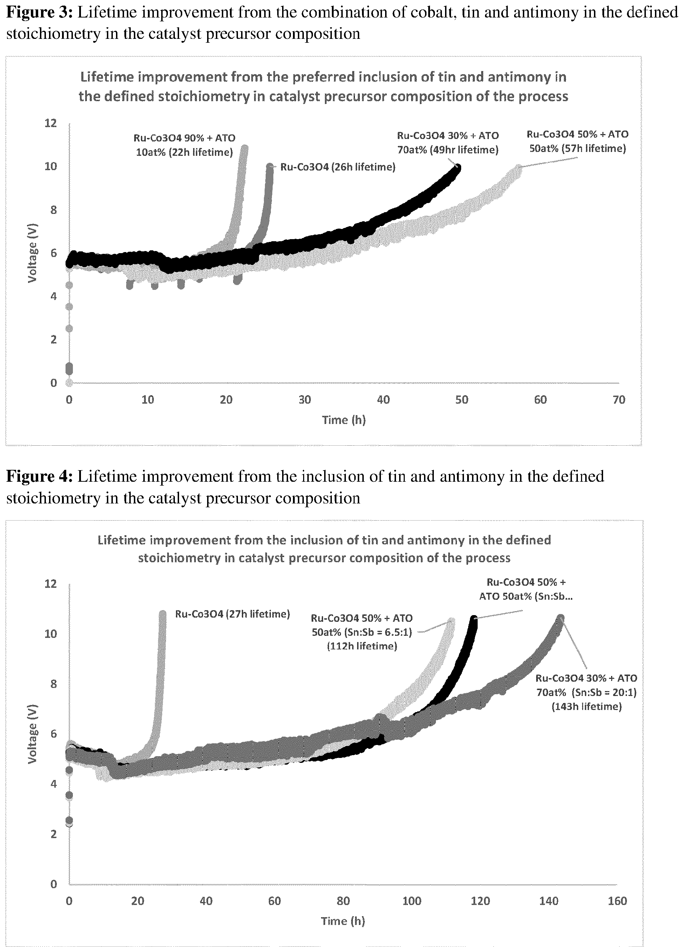

The advantageous presence of cobalt, tin and antimony in the specified stoichiometric ratios is illustrated in Figures 3 and 4. Analogous anodes were prepared according to the Embodiment 1 example in section 1 above, but using catalyst precursor compositions varying in their tin and antimony contents as indicated in the figure. In each case, lifetime testing was conducted and the (over)-potentials compared over time.

As Figure 3 demonstrates, the overall metallic stoichiometric ratio of the cobalt : total of antimony and tin in the catalyst precursor composition is influential on the lifetime of the anode, and anode structures made from a catalyst precursor composition wherein this molar ratio in the range of 20:80 to 80:20, and is preferably 50:50 show far improved durability in terms of maintaining low potentials for longer operating lives. The molar ratio of 50:50 provided best performance in this regard.

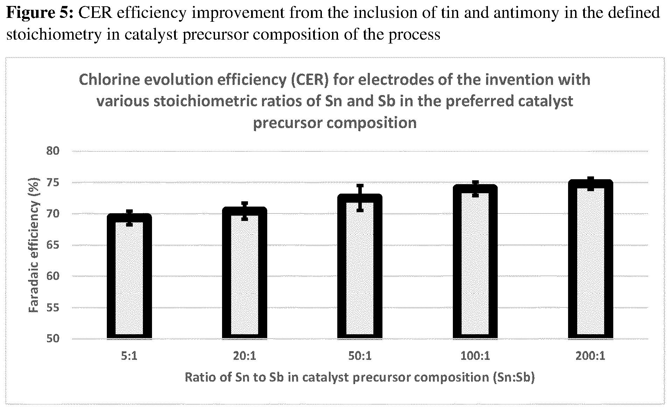

As Figure 4 demonstrates for analogous anodes prepared in line with the Embodiment 2 example in section 1 above, where the compounds of antimony and tin are varied, amounts providing a catalyst precursor composition with an overall metallic stoichiometric ratio of antimony : tin of at most 1:20 show a lifetime advantage over anodes with a ratio greater than 1: 10. In addition, in Figure 5, the CER efficiency as measured by the Faradic Efficiency (FE) using the method detailed earlier in this specification showed a growing advantage for the ratios 1:20, 1:50, 1:100 and 1:200, with the latter being the most highly effective catalyst precursor composition, resulting in an anode having the highest efficiency.

### 2.1(b) Inclusion of palladium oxide in the catalyst composition

In a further experiment, anodes were coated directly with a catalyst composition containing cobalt oxide particles, ruthenium oxide and tin and antimony oxides according to the invention, and the effect of introducing palladium as a further constituent evaluated. The results are shown in the table below, which demonstrates the improvement in efficiency brought about by the preferred inclusion of a small amount of palladium into the catalyst composition.

| Electrode type | Atomic ratio of metal elements (catalyst layer) (atom%) | Low Salt efficiency (%) |
|---|---|---|
| Ru-Co₃O₄-ATO 10 layer coating | Co: 69.2, Ru 7.7, Sn 23.0, Sb: 0.1 | 76.9 ± 0.6 |
| Ru-Co₃O₄-ATO + Pd 1at% 10 layer coating | Co: 68.8, Ru: 7.6, Sn: 22.8, Sb: 0.1, Pd: 0.7 | 83.7 ± 1.0 |

### (Exposed electrode area (Scm²), 1.17 g/L NaCl concentration)

2.1(c) Improvement provided by catalyst composition on anode structure with dimensionally-stable anode interlayer.

The CER Efficiency Test and CER Lifetime Test was measured for a conventional anode coated with a dimensionally stable anode composition (as measured by SEM/EDX with an acceleration voltage of 20 kV. Ru: 15.7, Ti: 11.93, Ir: 1.51 at%), in comparison with a modified version of the same anode onto which had been applied a ruthenium oxide intermediate layer, followed by a metallurgical catalyst composition, in accordance with Embodiment 3 in Section 1 above. The results are shown in the Table below:

| **Sample** | **CER Lifetime (hr)** | **CER Efficiency (%)** |
|---|---|---|
| Anode with conventional DSA layer only | 302 | 56.3 |
| Anode with conventional DSA layer, RuO2 intermediate layer, and catalyst layer of the invention | 403 | 72.4 |

As the results show, the addition of the cobalt-based metallurgical catalyst layer and ruthenium oxide intermediate layer to form the anode of the invention resulted in a CER efficiency increase of 16.1% over the conventional DSA catalyst layer alone; and a Lifetime improvement for the anode of the invention of 33% over the conventional anode.

## Claims

1. A catalytic anode structure for use in an electro-chlorination system comprising an electrically conductive solid substrate overlaid, on its electrolyte-facing surface, with an electrically-conductive metallurgical catalyst layer, this layer comprising cobalt oxide crystalline particles consisting essentially of crystalline cobalt oxide having the chemical formula Co₃O₄, within a crystalline matrix of oxides of antimony and tin;
wherein the cobalt oxide is present in an amount of at least 12.5 atom% of the total metal atom content of the metallurgical catalyst layer; and wherein the overall metallic stoichiometric ratio of the cobalt : total of antimony and tin in the metallurgical catalyst layer is in the range of 2:1 to 9:1.

2. The anode structure of claim 1, wherein the compounds of antimony and tin are present in amounts providing the metallurgical catalyst layer with an overall metallic stoichiometric ratio of antimony : tin in the range of 1:5 to 1:200, and preferably in the range of 1:5 to 1:100.

3. The anode structure of claim 1 or claim 2, wherein the metallurgical catalyst layer further comprises one or more noble metals or compound(s) thereof, preferably ruthenium or an oxide thereof.

4. The anode structure of claim 3, wherein the cobalt oxide crystalline particles in the metallurgical catalyst layer consist essentially of ruthenium or an oxide thereof to the level of at most 5 atom% of ruthenium based on the total metal atom content of the crystalline particles, at least some of said ruthenium being present in the form of surface decoration on the crystalline particles.

5. The anode structure of claim 4, wherein the cobalt oxide crystalline particles in the metallurgical catalyst layer further contain crystalline regions wherein ruthenium is incorporated within the crystal lattice of the cobalt oxide, or has formed a mixed metal oxide with cobalt, or both.

6. The anode structure of any of claims 3 to 5 wherein the metallurgical catalyst layer comprises at least one compound of palladium in an amount providing the metallurgical catalyst layer with an overall palladium content of at most 7 atom% of the total metal atom content of the metallurgical catalyst layer, and preferably at most 3 atom%.

7. The anode structure of any of claims 3 to 6 wherein the metallurgical catalyst layer additionally comprises ruthenium oxide distributed throughout, or substantially throughout, the catalyst layer.

8. The anode structure of any of claims 3 to 7 wherein the metallurgical catalyst layer additionally comprises iridium, in the form of its oxide or other catalytically active species, in an amount providing an overall iridium content of at most 15 atom% of the total metal atom content of the metallurgical catalyst layer, and preferably at most 5 atom%.

9. The anode structure of any of claims 1 to 8, wherein the electrically conductive solid substrate consists essentially of a valve metal, preferably titanium.

10. The anode structure of any of claims 1 to 9 wherein the metallurgical catalyst layer additionally comprises at least one compound of copper in an amount providing the layer with an overall copper content of at most 10 atom%, and preferably at most 2 atom% of the metallurgical layer; or at least one compound of aluminium in an amount providing the layer with an overall aluminium content of at most 30 atom%, and preferably at most 20 atom% of the metallurgical layer; or at least one compound of nickel in an amount providing the layer with an overall nickel content of at most 15 atom%, and preferably at most 10 atom%, of the total metal atom content of the metallurgical catalyst layer; or more than one of the above.

11. The anode structure of any of claims 1 to 10 wherein the metallurgical catalyst layer does not comprise titanium or a compound thereof.

12. The anode structure of any of claims 1 to 11 wherein the anode structure consists of an electrically conductive solid substrate overlaid, on its electrolyte-facing surface, with the electrically conductive metallurgical catalyst layer defined in any of claims 1 to 11, the catalyst layer lying directly adjacent to the substrate.

13. The anode structure of any of claims 1 to 11 wherein the anode structure comprises an electrically conductive solid substrate overlaid, on its electrolyte-facing surface, with a metallurgical interlayer lying directly adjacent to the substrate; the interlayer being overlaid by the electrically conductive metallurgical catalyst layer defined in any of claims 1 to 11.

14. The anode structure of claim 13, wherein the interlayer is a dimensionally stable anode composition.

15. The anode structure of claim 13 or claim 14, wherein the interlayer comprises titanium or one or more compounds thereof, or tantalum or one or more compounds thereof, or both.

16. The anode structure of any of claims 13 to 15, wherein the interlayer comprises ruthenium or one or more compounds thereof, and preferably also titanium or one or more compounds thereof.

17. The anode structure of any of claims 13 to 16, wherein the interlayer comprises iridium or one or more compounds thereof.

18. The anode structure of any of claims 13 to 17 wherein the titanium, tantalum, ruthenium or iridium compounds where present comprise oxide compounds, and preferably consist of oxide compounds.

19. A process for the preparation of a catalytic anode structure for use in an electro-chlorination system, comprising the following sequential steps:
a) the preparation or obtention of an electrically conductive solid substrate;
b) the application, to the electrolyte-facing surface of the substrate, of an electrically-conductive metallurgical catalyst precursor composition, being a composition comprising cobalt oxide crystalline particles consisting essentially of crystalline cobalt oxide having the chemical formula C_{O3}O₄ dispersed in a mixture of compounds of antimony and tin, wherein the cobalt oxide is present in an amount of at least 12.5 atom% of the total metal atom content of the composition, and wherein the overall metallic stoichiometric ratio of the cobalt: total of antimony and tin in the composition is in the range of 20:80 to 80:20;
wherein the process solvent used for applying the metallurgical catalyst precursor composition in step (b) is a mixture of isopropanol and water in the ratio range of 80:20 to 95:5 by volume; and
wherein the resulting structure is thereafter dried, and then heat-treated at a temperature of at least 450°C.

20. The process of claim 19, wherein the substrate (a) is heated to a temperature in the range of 55-200°C during step (b), and the resulting structure thereafter dried at a temperature within the same range.

21. The process of claim 20 wherein the substrate (a) is heated to a temperature of 100°C throughout step (b), and the resulting structure thereafter dried at that temperature.

22. The process of any of claims 19 to 21 wherein, after drying, the heat treatment temperature is in the range of 450 to 550°C.

23. The process of any of claims 19 to 22, wherein the heat treatment temperature is 500°C.

24. The process of any of claims 19 to 23, wherein the process solvent used for applying the metallurgical composition in step (b), is a mixture of isopropanol and water in the ratio range of 85:15 by volume.

25. The process of any of claims 19 to 24, wherein each metallurgical composition applied in step (b) is sequentially applied by brush-coating in one or more passes over the underlying structure.

26. The process of any of claims 19 to 25, wherein each metallurgical composition applied in step (b) is sequentially applied by spray-coating in one or more passes over the underlying structure.

27. The process of any of claims 19 to 26, wherein the cobalt oxide crystalline particles in the metallurgical catalyst precursor composition further consist essentially of ruthenium to the level of at most 5 atom % of ruthenium based on the total metal atom content of the crystalline particles, at least some of said ruthenium being present in the form of surface decoration on the crystalline particles.

28. The process of claim 27, wherein the cobalt oxide crystalline particles in the metallurgical catalyst precursor composition further contain crystalline regions wherein some ruthenium has become incorporated within the crystal lattice of the cobalt oxide, or has formed a mixed metal oxide with cobalt, or both.

29. The process of any of claims 19 to 28 wherein the compounds of antimony and tin are present in amounts providing the metallurgical catalyst precursor composition with an overall metallic stoichiometric ratio of antimony : tin of at most 1: 10, and preferably of at most 1:20; and most preferably of 1:200.

30. The process of any of claims 19 to 29 wherein the metallurgical catalyst precursor composition additionally comprises at least one compound of palladium, in an amount providing the composition with an overall palladium content of preferably at most 7 atom%, and preferably at most 5 atom%, based on the total metal atom content of composition.

31. The process of any of claims 19 to 30, wherein the tin and antimony compounds in the metallurgical catalyst precursor composition each comprise compounds other than oxide compounds; and wherein their heated application in step (b), followed by drying and heat treatment, thermally decomposes these compounds into one or more of their respective metal oxide or mixed metal oxide compounds within the metallurgical catalyst layer of the final anode structure.

32. The process of claim 31, wherein the tin and antimony compounds where present initially comprise, and preferably consist of, their respective halide or sulfate compounds.

33. The process of any of claims 19 to 32 wherein the metallurgical catalyst precursor composition additionally comprises at least one compound of copper, or at least one compound of aluminium, or at least one compound of nickel, or more than one of the above; in amounts respectively providing the composition with an overall copper content of at most 10 atom% , and preferably at most 2 atom% based on the total metal atom content of the composition , or an overall aluminium content of at most 30 atom%, and preferably at most 20 atom% based on the total metal atom content of the composition, or an overall nickel content of at most 15 atom%, and preferably at most 10 atom% based on the total metal atom content of the composition, ormore than one of the above.

34. The process of any of claims 19 to 34 wherein the metallurgical catalyst precursor composition additionally comprises at least one compound of iridium, in an amount respectively providing an overall iridium content of at most 15 atom%, and preferably at most 10 atom%, based on the total metal atom content of that composition.

35. The process of any of claims 19 to 34, wherein the electrically conductive solid substrate consists essentially of a valve metal, preferably titanium.

36. The process of any of claims 19 to 35 wherein, in step (b), the metallurgical catalyst precursor composition is applied directly to the electrolyte-facing surface of the substrate.

37. The process of any of claims 19 to 36 wherein, prior to step (b), a metallurgical base composition is applied directly to the electrolyte-facing surface of the substrate to form an metallurgical interlayer directly adjacent to the substrate surface; and wherein in step (b) the metallurgical catalyst precursor composition is applied to the outer surface of the metallurgical interlayer.

38. An electro-chlorination system comprising the electrolytic anode structure of any of claims 1 to 18 or obtainable by the process of any of claims 19 to 37.

39. An electrolytic process for the evolution of chlorine gas from an aqueous solution containing chloride ions, which comprises passing an electrical current through said solution in an electrolytic cell comprising the electrolytic anode structure of any of claims 1 to 18 or obtainable by the process of any of claims 19 to 37, wherein chlorine gas is evolved from the aqueous solution at the anode.

40. The electrolytic process of claim 39, wherein chlorine gas evolution from the aqueous solution is promoted by the catalytic action of the metallurgical catalyst layer overlaying the surface of the anode.

41. The electrolytic process of claim 39 or 40, wherein the chlorine gas evolved by the process is used as a biocide to disinfect water.

42. The electrolytic process of claim 41, wherein the chlorine gas is used to disinfect ballast water or industrial waste water.

43. The electrolytic process of claim 39 or 40, wherein the process is a chlor-alkali process for the generation and recovery of chlorine.
